(19) Europäisches Patentamt
European Patent Office
Office européen des brevets

(11) Veröffentlichungsnummer : **0 036 387**
**B1**

(12) # EUROPÄISCHE PATENTSCHRIFT

(45) Veröffentlichungstag der Patentschrift : **22.01.86**

(51) Int. Cl.⁴ : **C 07 D209/44**, C 09 B 57/04

(21) Anmeldenummer : **81810080.2**

(22) Anmeldetag : **09.03.81**

(54) **Verfahren zur Herstellung von Hydrazono-isoindolinen.**

(30) Priorität : **13.03.80 CH 1974/80**

(43) Veröffentlichungstag der Anmeldung : **23.09.81 Patentblatt 81/38**

(45) Bekanntmachung des Hinweises auf die Patenterteilung : **22.01.86 Patentblatt 86/04**

(84) Benannte Vertragsstaaten : **CH DE FR GB IT LI**

(56) Entgegenhaltungen :
**DE-B- 1 670 748**
**US-A- 4 132 708**
**JOURNAL OF AMERICAN CHEMICAL SOCIETY, Band 56, Oktober 1934 G.E.P. SMITH et al.: "The Ammono Ketone-Alcohols. I. Benzophenoneimine", Seiten 2095-2098.**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Iqbal, Abul, Dr.**
**Im Schaiengarten 1**
**CH-4107 Ettingen (CH)**
Erfinder : **Lienhard, Paul, Dr.**
**Kirschgartenstrasse 14**
**CH-4402 Frenkendorf (CH)**

**EP 0 036 387 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Hydrazono-isoindoline der Formel

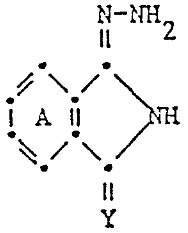

(I)

sind wertvolle Zwischenprodukte für die Herstellung von Farbstoffen und Pigmenten. Man erhält sie u. a. durch Umsetzen eines Iminoisoindolins der Formel

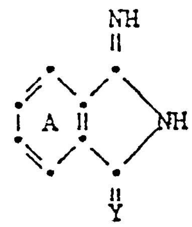

(II)

mit Hydrazinhydrat. Nach heutigem Stand der Technik sind für diesen Reaktionsschritt höhere Temperaturen von etwa 80 bis etwa 220 °C erforderlich. So beschreibt z. B. DE-OS 1 670 748 (Bsp. 79) die Umsetzung des 1-(Cyanmethoxycarbonyl-methylen)-3-imino-isoindolins mit Hydrazinhydrat in siedendem Eisessig. Allerdings führen solche Reaktionsbedingungen zu unbefriedigenden Ergebnissen. Zwar entsteht ein Reaktionsprodukt in guter Ausbeute. Es besteht jedoch nur zum Teil aus der gewünschten Hydrazono-Verbindung. Diese lässt sich aus dem Gemisch nur sehr schwer in reiner Form gewinnen. Die effektive Ausbeute an gewünschtem Produkt ist nur ein Bruchteil der für das Gemisch angegebenen Zahl und für einen technischen Prozess ungenügend.

Es wurde nun gefunden, dass man zu 1-Hydrazonoisoindolinen der Formel I gelangt, worin Y

a) einen Methinrest der Formel

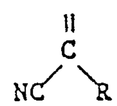

bedeutet, worin R für eine Alkoxycarbonyl-, Alkylcarbamoyl-, Carbamoyl- oder Sulfamoylgruppe, eine Benzylcarbamoylgruppe, oder eine Gruppe der Formel CONHB bedeutet, worin B einen Benzolrest bedeutet, der Halogenatome, Alkyl- oder Alkoxygruppen mit 1-6 C, Alkanoylaminogruppen mit 2-6 C, Carbamoyl oder Trifluormethylgruppen, oder eine gegebenenfalls durch Halogenatome oder Alkylgruppen mit 1-4 C substituierte Phenoxy- oder Benzoylaminogruppe aufweisen kann, oder worin R für eine gegebenenfalls durch Chloratome substituierte Naphthylcarbamoylgruppe, eine Phenylsulfonylgruppe oder einen Rest der Formel

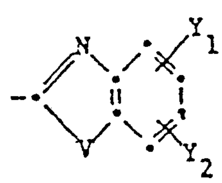

steht, worin V ein O- oder S-Atom oder eine Iminogruppe, $Y_1$ und $Y_2$ H- oder Halogenatome, Alkyl- oder Alkoxygruppen mit 1-4 C oder Nitrogruppen bedeuten oder b) einen sich von einer heterocyclischen Verbindung mit aktiver Methylengruppe ableitenden Methinrest oder c) einen sich von einem heterocyclischen aromatischen Amin ableitenden Iminrest bedeutet, und der Ring A nicht-wasserlöslichmachende Substituenten aufweisen kann, durch Umsetzung eines Iminoisoindolins der Formel

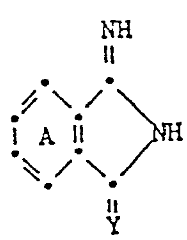

worin A und Y die oben angegebenen Bedeutungen haben, mit Hydrazin oder einem Hydrazin abgebenden Mittel in einem polaren Lösungsmittel, wenn man die Umsetzung bei Temperaturen von höchstens 40° durchführt und das Reaktionsgemisch, sofern es eine aliphatische Carbonsäure enthält, letztere in nicht mehr als der doppelt molaren Menge, bezogen auf das Iminoisoindolin, vorhanden ist.

Die oben erwähnten Ausgangsstoffe erhält man durch Kondensation des entsprechenden 1-Amino-3-imino-isoindolenins mit einem Acetonitril der Formel $NC-CH_2-R$ im Molverhältnis 1 : 1. Als Beispiel seien die in der GB-PS 1, 467, 595, Seite 7 aufgeführten Acetonitrile erwähnt, ausserdem Cyanessigsäure-o-chlorphenyl-, p-chlorphenyl-, -m-chlorphenyl-, -m-methylphenyl-, -p-methylphenyl-, -3,4-dichlorphenyl-, -3,5-dimethylphenyl-, -3,4-dimethylphenyl, -3-chlor-4-methylphenyl-, -o-methoxyphenyl-, -2,4-dimethoxyphenyl-, -2,5-dimethoxyphenyl-, p-acetylamino-phenyl-, p-benzoylaminophenyl-, -3-chlor-4-methyl-, p-chlorbenzoylaminophenyl, -4-carbamoylphenyl-, -4-sulfamoylphenyl-, -4-phenylazophenyl-, -4-phenoxyphenyl-, -p-nitrophenyl-, -3-trifluormethylphenyl-, oder -2-chlor-5-trifluormethyl-phenylamide, 2-Cyanmethyl-4-phenyl-, -4-p-nitrophenyl-, -4-fluorphenyl- oder -4-methylphenylthiazol.

Bevorzugte Ausgangsprodukte sind Imino-isoindoline der Formel

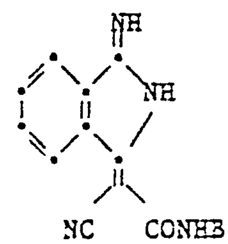

worin B einen Benzolrest bedeutet, der Halogenatome, Alkyl- oder Alkoxygruppen mit 1-6 C, Alkanoylaminogruppen mit 2-6 C eine Carbamoyl- oder Trifluormethylgruppe oder eine gegebenenfalls durch Halogenatome oder Alkylgruppen mit 1-4 C substituierte Phenoxy- oder Benzoylaminogruppen aufweisen kann.

Die Umsetzung des Iminoisoindolins mit dem Hydrazin erfolgt in einem polaren Lösungsmittel insbesondere einem solchen hydrophiler Natur, beispielsweise einem Amid wie Dimethylformamid, Formamid, Dimethylacetamid oder N-Methylpyrrolidon, ferner Dimethyl-sulfoxid, Acetonitril oder einem Alkohol wie beispielsweise Aethylcellosolve. Es kann auch ein Gemisch polarer Lösungsmittel verwendet werden. Dieses kann zur Neutralisation des bei der Kondensation freigesetztem Ammoniaks eine aliphatische Carbonsäure, wie Eisessig, in höchstens der doppelten molaren Menge bezogen auf das Iminoisoindolin enthalten. Man arbeitet jedoch vorzugsweise in Abwesenheit aliphatischer Carbonsäuren.

Die Umsetzung liefert ein besonders reines Produkt, wenn stöchiometrische Mengen des Hydrazins respektiv der Hydrazin abgebenden Verbindung eingesetzt werden.

Die Umsetzungstemperatur liegt zweckmässig zwischen 10-40°.

Die Isolierung des erhaltenen Hydrazono-isoindolins erfolgt zweckmässig durch Zugabe eines aliphatischen Alkohols mit 1-3 C, insbesondere Aethanol, wobei das Endprodukt ausfällt und durch Filtrieren abgetrennt werden kann. Es wird in ausgezeichneter Ausbeute und Reinheit erhalten und kann ohne weitere Reinigung als Ausgangsprodukt für weitere Synthesen, insbesondere für die Kondensation mit Aldehyden oder Ketonen gemäss GB-PS 1 467 595 zur Herstellung von Pigmenten verwendet werden. Es kann aber auch ohne Zwischenisolierung in einer Eintopfreaktion zu Pigmenten weiterverarbeitet werden. Dank der Reinheit des erfindungsgemäss erhältlichen Hydrazono-isoindolins werden die daraus hergestellten Pigmente in guter Ausbeute und Reinheit erhalten.

## Beispiel 1

11,6 g 1,3-Diiminoisoindolin 75-proz. (0,06 Mol) und 11,7 g Cyanacet-p-chloranilid (0,06 Mol) werden in 50 ml Dimethylformamid und 3,6 ml (0,06 Mol) Eisessig gelöst und über Nacht bei Raumtemperatur (20-23 °C) gerührt. Eine Dünnschichtchromatographische Kontrolle zeigt am nächsten Tag vollständige Umsetzung zum 1-(Cyano-p-chlorphenylcarbamoylmethylen)-3-imino-isoindolin an. Zu der erhaltenen Suspension lässt man hierauf bei Raumtemperatur innert 1-2 Minuten 3,1 ml Hydrazinhydrat (0,06 Mol) zutropfen. Das Gemisch wird bei Raumtemperatur 45 Minuten gerührt. Durch anschliessende dünnschichtchromatographische Untersuchung lässt sich vergewissern, dass sich das 1-(Cyano-p-chlorphenylcarbamoylmethylen)-3-imino-isoindolin vollständig umgesetzt hat, worauf man das Gemisch mit 250 ml Aethanol versetzt und bei Raumtemperatur 15-20 Minuten gut rührt. Die Fällung wird filtriert, mit wenig Aethanol gewaschen und über Nacht bei 50-60 °C unter Vakuum getrocknet. Man erhält 16,8 g (83 % d. Theorie) der Verbindung der Formel

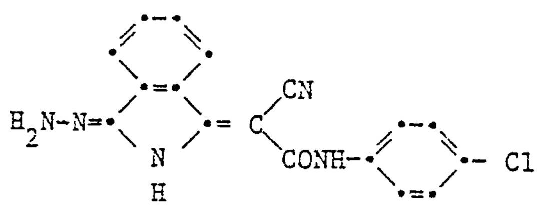

als gelbbraunes Pulver.

Mikroanalyse : $C_{17}H_{12}ClN_5O$ MG 338
ber. : 60,45 % C 3,58 % H 20,74 % N 10,50 % Cl
gef. : 60,6 % C 3,9 % H 20,7 % N 10,5 % Cl

Beispiel 1B

11,6 g 1,3-Diiminoisoindolin 75-proz. (0,06 Mol) und 11,7 g Cyanacet-p-chloranilid (0,06 Mol) werden in 50 ml Dimethylformamid und 3,6 ml (0,06 Mol) Eisessig gelöst und über Nacht bei Raumtemperatur (20-23 °C) gerührt. Eine dünnschichtchromatographische Kontrolle zeigt am nächsten Tag vollständige Umsetzung zum 1-(Cyano-p-chorphenylcarbamoylmethylen)-3-iminoisoindolin an. Man versetzt die erhaltene Suspension innert 1-2 Minuten mit 3,1 ml Hydrazinhydrat (0,06 Mol), erhitzt auf 100 °C und lässt das Gemisch bei derselben Temperatur während 45 Minuten ausreagieren. Hierauf kühlt man auf Raumtemperatur ab, gibt 250 ml Aethanol zu und rührt das Gemisch weitere 15-20 Minuten bei Raumtemperatur. Die Fällung wird anschliessend filtriert, mit wenig Aethanol gewaschen und über Nacht bei 50-60 °C unter Vakuum getrocknet. Man erhält 15,8 g (78 % d. Theorie) einer braunen Verbindung, die praktisch die gleichen mikroanalytischen Werte wie diejenigen der analog bei Raumtemperatur nach Beispiel 1 erhaltenen Verbindung aufweist.

Mikroanalyse : $C_{17}H_{12}ClN_5O$ Mol.-Gew. 338
ber. : 60,45 % C 3,58 % H 20,74 % N 10,50 % Cl
gef. : 60,9 % C 3,4 % H 19,6 % N 10,2 % Cl

Beispiel 2

19,3 g 1,3-Diiminoisoindolin 75-proz. (0,1 Mol) und 16,02 g Cyanacetanilid wurden in 150 ml Dimethylformamid und 6 ml (0,1 Mol) Eisessig gelöst und über Nacht bei Raumtemperatur (20-23 °C) gerührt. Am nächsten Tag tropft man zu der gebildeten gelben Suspension bei Raumtemperatur innert 2-3 Minuten 5,2 ml (0,105 Mol) Hydrazinhydrat zu und rührt bei Raumtemperatur 45 Minuten lang. Hierauf wird das Gemisch mit 1 000 ml Aethanol versetzt und bei Raumtemperatur 15-20 Minuten tüchtig gerührt. Die Fällung wird filtriert, mit wenig Aethanol gewaschen und über Nacht bei 50-60 °C unter Vakuum getrocknet. Man erhält 27,2 g (89,7 % der Theorie) der Verbindung der Formel

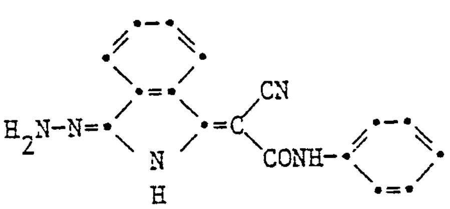

als gelbes Pulver.

Mikroanalyse
ber.** : 66,97 % C 4,36 % H 22,97 % N
gef. : 67,0 % C 4,3 % H 23,3 % N 0,5 % $H_2O$

Bei analoger Arbeitsweise, aber Verwendung von Aethylcellosolve, Formamid, Dimethylacetamid, Pyrrolidon, N-Methyl-pyrrolidon oder Acetonitril anstele von Dimethylformamid erhält man das gleiche Produkt in ebenfalls guter Ausbeute.

Beispiel 3

Arbeitet man analog Beispiel 2 mit Cyanacet-p-toluidid anstatt Cyanacetanilid unter sonst gleichen

** unter Berücksichtigung der gefundenen Wassermenge von 0,5 %.

Bedingungen, so erhält man nach üblicher Aufarbeitung 27 g (85 % d. Theorie) der Verbindung der Formel

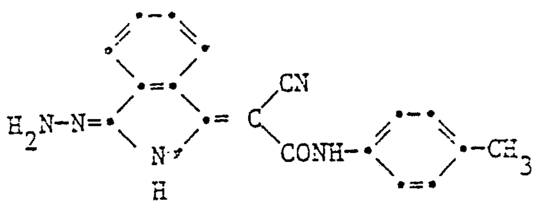

als braungelbes Pulver.

Mikroanalyse

| | | | |
|---|---|---|---|
| ber. : | 68,13 % C | 4,77 % H | 22,07 % N |
| gef. : | 67,9 % C | 4,8 % H | 22,1 % N |

Beispiele 4-6

Nach dem den Beispielen 1-3 zugrundeliegenden Verfahren wurden weitere, in Tab. 1 zusammengestellte Hydrazone der Struktur

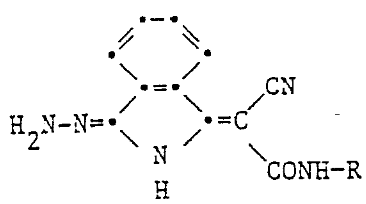

hergestellt.

(Siehe Tabelle 1 Seite 6 f.)

Tabelle 1

| Beispiel Nr. | R | Ausbeute % | Mikroanalyse ber. % C | H | N | gef. % C | H | N |
|---|---|---|---|---|---|---|---|---|
| 4 | 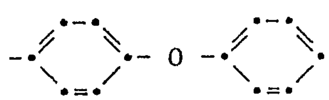 | 84 | 69,86 | 4,3 | 17,7 | 69,2 | 4,5 | 17,6 |
| 5 | 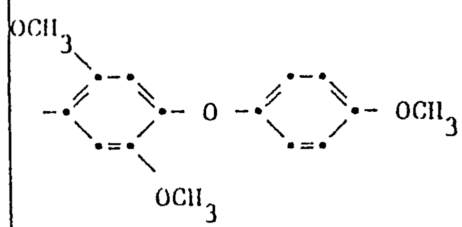 | 77 | 64,2 | 4,97 | 14,4 | 64,2 | 4,9 | 14,5 |
| 6 | 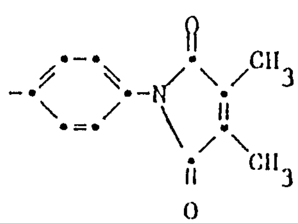 | 67 | *62,8 | *4,4 | *19,1 | 62,5 | 4,7 | 19,0 |

* unter Berücksichtigung der gefundenen Wassermenge von 2,8 %.

Beispiel 7

9,8 g (0,034 Mol) 1-(Cyano-phenylcarbamoylmethylen)-3-iminoisoindolin, aus 1,3-Iminoisoindolin und Cyanacetanilid hergestellt, werden in 95 ml Pyrrolidon-2 bei 80-90 °C gelöst und anschliessend auf 15 °C gekühlt. Hierauf gibt man innert 1-2 Minuten 1,88 ml (0,039 Mol) Hydrazinhydrat zu. Das Reaktionsgemisch wird bei Raumtemperatur (20-23 °C) 45 Minuten lang gerührt. Anschliessend gibt man 200 ml Methanol zu und lässt 15-20 Minuten bei derselben Temperatur rühren, kühlt danach auf 10 °C ab, filtriert und wäscht das Nutschgut mit wenig Methanol nach. Nach dem Trocknen bei 60-70 °C unter Vakuum erhält man 6,55 g (63,5 % d. Theorie) einer Verbindung, die mit dem nach Beispiel 2 hergestellten Zwischenprodukt chemisch identisch ist.

Beispiel 8

32,2 g (0,1 Mol) 1-(Cyano-p-chrophenylcarbamoyl-methylen)-3-imino-isoindolin, aus 1,3-Diiminoisoindolin und Cyanacet-p-chloranilid hergestellt, werden in 150 ml Dimethylformamid gut angeschlämmt. Man tropft bei Raumtemperatur innert 2-3 Minuten 5,2 ml (0,106 Mol) Hydrazinhydrat zu und rührt das Gemisch bei derselben Temperatur 45 Minuten. Hierauf wird es mit 1 000 ml Aethanol versetzt und bei Raumtemperatur weitere 15-20 Minuten gut gerührt. Die gebildete Fällung wird abfiltriert, mit Aethanol nachgewaschen und über Nacht bei 50-60 °C unter Vakuum getrocknet. Man erhält 28,7 g (85 % d. Theorie) einer Verbindung mit der gleichen Zusammensetzung wie im Beispiel 1.

Beispiel 9

6,24 g (0,02 Mol) 1-p-Chlorphenyl-3-methyl-4-anilino-methylen-5-pyrazolon und 5,2 g (0,020 4 Mol) Nickelacetat. 4 $H_2O$ werden in 80 ml Dimethylformamid bei 60-70 °C gelöst. Anschliessend gibt man unter Rühren 6,7 g (0,02 Mol) des nach Beispiel 1 erhaltenen 1-(Cyano-p-chlorphenylcarbamoylmethylen)-3-hydrazino-isoindolins zu. Das Gemisch wird auf 115-120 °C erhitzt und bei derselben Temperatur während 2 Stdn. weitergerührt. Danach wird auf 80 °C abgekühlt und der resultierende Metallkomplex abfiltriert. Das Filtergut wird mit Dimethylformamid und Aethanol gut gewaschen und bei 80 °C unter Vakuum getrocknet. Man erhält 9,7 g (79 % der Theorie) eines roten Nickelkomplexes der Zusammensetzung $C_{28}H_{17}Cl_2N_7NiO_2$ und der Formel (nur eine der möglichen isomeren bzw. tautomeren Formen wurde berücksichtigt)

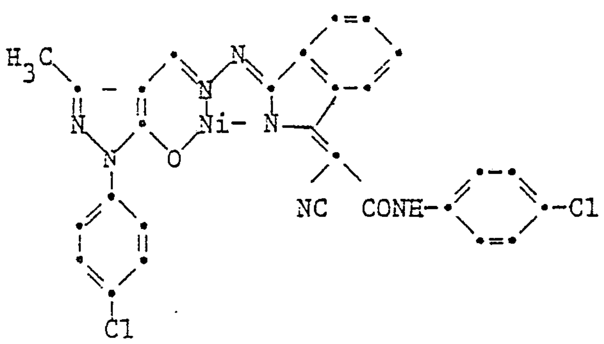

Mikroanalyse : $C_{28}H_{17}Cl_2N_7NiO_2$ Mol.-Gew. 613

| | | | | | |
|---|---|---|---|---|---|
| ber. : | 54,85 % C | 2,80 % H | 15,99 % N | 11,57 % Cl | 9,58 % Ni |
| gef. : | 54,80 % C | 3,1 % H | 15,90 % N | 11,40 % Cl | 9,46 % Ni |

Der obige 1 : 1 $Ni^{+2}$-Komplex färbt PVC, Kunststoffe und Lacke in reinen scharlachroten Tönen von ausgezeichneter Licht-, Wetter-, Hitze- und Migrationsechtheit.

Beispiel 10

Verwendet man im Beispiel 9 anstelle des nach Beispiel 1 erhaltenen 1-(Cyano-p-chlorphenylcarbamoylmethylen)-3-hydrazino-isoindolins das aus Beispiel 1B stammende Zwischenprodukt, so erhält man unter sonst identischen Reaktionsbedingungen nunmehr lediglich 3,1 g (25,3 % d. Theorie) eines ebenfalls roten Nickelkomplexes mit der gleichen Zusammensetzung wie im Beispiel 9.

Mikroanalyse : $C_{28}H_{17}Cl_2N_7NiO_2$ Mol.-Gew. 613

| | | | | | | |
|---|---|---|---|---|---|---|
| ber.* : | 54,49 % C | 2,85 % H | 15,89 % N | 11,49 % Cl | 9,51 % Ni | |
| gef. : | 54,0 % C | 2,9 % H | 16,0 % N | 11,1 % Cl | 9,86 % Ni | 0,7 % $H_2O$ |

* unter Berücksichtigung der gefundenen Wassermenge von 0,7 %.

Allerdings färbt der vorliegende Metallkomplex PVC und Lacke in nicht mehr so reinen bzw. so licht- und wetterechten Tönen wie das Metallkomplex-Pigment vom Beispiel 9.

Ein Vergleich der Ausbeute sowie Echtheiten der nach Beispiel 9 und 10 hergestellten Metallkomplex-Pigmente beweist damit eindeutig, dass für die Herstellung von Metallkomplex-Zwischenstufen vom Typ 1-Methylen-3-hydrazono-isoindolin das erfindungsgemässe, vorzugsweise bei Raumtemperatur (20-40 °C) ausgeführte Verfahren nach Beispiel 1 gegenüber dem in der DOS 1 670 748 beschriebenen Verfahren 1B, wesentliche Vorteile bietet.

Beispiel 11

Zu einer Mischung von 7,17 g (0,022 Mol) 1-(Cyan-p-chlorphenylcarbamoylmethylen)-3-imino-isoindolin, hergestellt aus 1,3-Diiminoisoindolin und Cyanessigsäure-p-chloranilid, in 50 ml Dimethylformamid lässt man bei Raumtemperatur innert 1-2 Minuten 1,1 ml (0,023 Mol) Hydrazinhydrat zutropfen. Das Gemisch wird bei Raumtemperatur 40-45 Minuten gerührt. Anschliessende dünnschichtchromatographische Kontrolle zeigt vollständige Umsetzung des 1-(Cyan-p-chlorphenylcarbamoyl-methylen)-3-imino-isoindolins an. Anschliessend wird mit einer 75-80 °C heissen Suspension von 6,86 g (0,022 Mol) 1-p-Chlorphenyl-3-methyl-4-anilinomethylen-5-pyrazolon und 5,75 g (0,023 Mol) Nickelacetat. 4 $H_2O$ in 25 ml DMF versetzt. Die Mischung wird auf 115-120 °C erhitzt und bei derselben Temperatur während 2 Stdn. weitergerührt. Danach wird auf 80 °C abgekühlt und der resultierende Metallkomplex abfiltriert. Das Filtergut wird mit Dimethylformamid und Aethanol gut gewaschen und bei 80 °C unter Vakuum getrocknet. Man erhält 10,8 g (80 % der Theorie) eines roten Nickelkomplexes mit gleicher Zusammensetzung und Pigmenteigenschaften wie im Beispiel 9.

Nach dem Eintopfverfahren des Beispiels 11 werden die Metallkomplexe untenstehender Formel (nur eine der möglichen tautomeren bzw. isomeren Formen wurde berücksichtigt) synthetisiert,

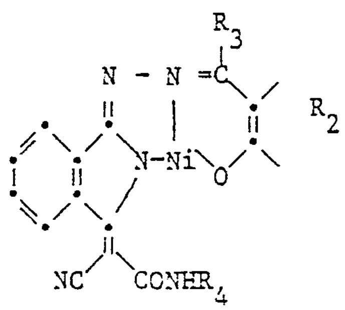

wobei $R_2$, $R_3$ und $R_4$ die in der Tabelle 2 angegebene Bedeutung haben. Die Ausgangsprodukte sind zum Teil bekannt oder nach bekannten Methoden herstellbar.

Tabelle 2

| Beispiel Nr. | $R_2$ | $R_3$ | $R_4$ | Nuance |
|---|---|---|---|---|
| 12 | 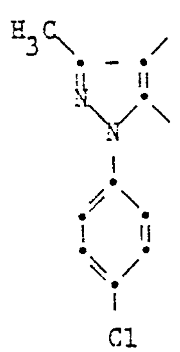 | H | 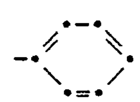 | scharlach |
| 13 | - do - | $CH_3$ | - do - | orange |

(Fortsetzung)

| Beispiel Nr. | $R_2$ | $R_3$ | $R_4$ | Nuance |
|---|---|---|---|---|
| 14 | 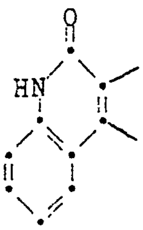 | H | 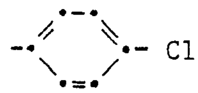 | rot |
| 15 | - do - | - do - | 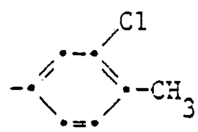 | orange |
| 16 | 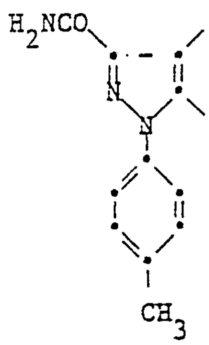 | H | 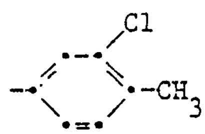 | rot |
| 17 | - do - | - do - | 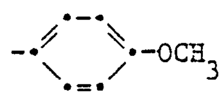 | scharlach |
| 18 | 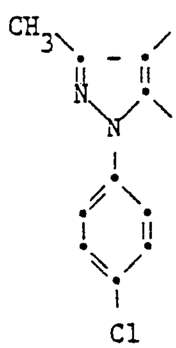 | - do - | 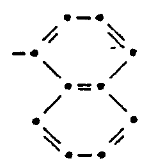 | orange |
| 19 | 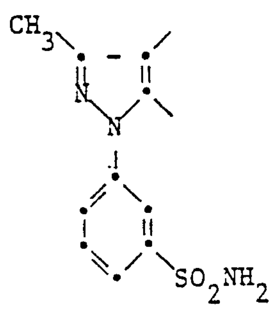 | - do - | 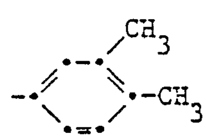 | |

(Fortsetzung)

| Beispiel Nr. | $R_2$ | $R_3$ | $R_4$ | Nuance |
|---|---|---|---|---|
| 20 | 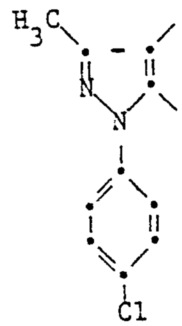 | H | 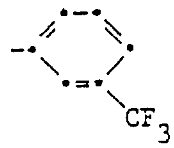 | orange |
| 21 | 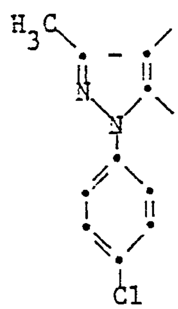 | H | 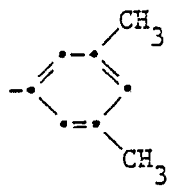 | rot |
| 22 | - do - | - do - | 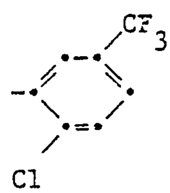 | rot |
| 23 | 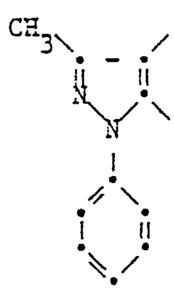 | - do - | 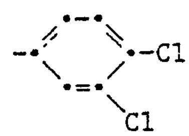 | orange |
| 24 | 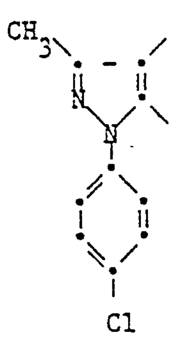 | - do - | 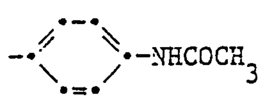 | rot |

(Fortsetzung)

| Beispiel Nr. | $R_2$ | $R_3$ | $R_4$ | Nuance |
|---|---|---|---|---|
| 25 | 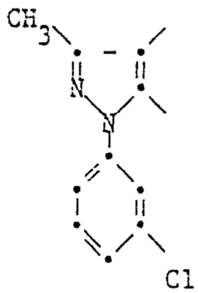 | - do - | 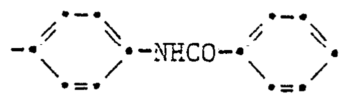 | orange-rot |
| 26 | 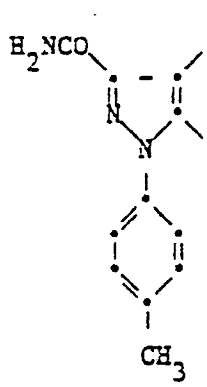 | $-CH_3$ | 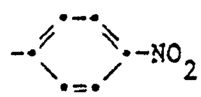 | orange |
| 27 | 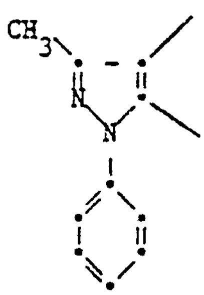 | - H - | 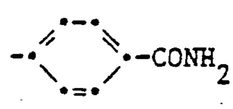 | rot |
| 28 | 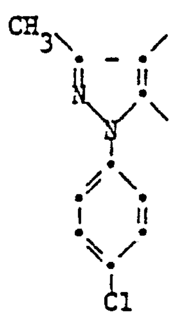 | - H - | 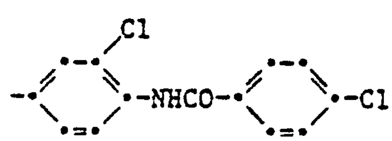 | orange |

(Fortsetzung)

| Beispiel Nr. | $R_2$ | $R_3$ | $R_4$ | Nuance |
|---|---|---|---|---|
| 29 | 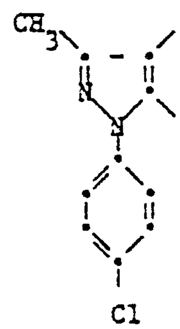 | H | 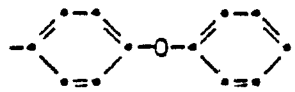 | scharlach |
| 30 | - do - | - do - | 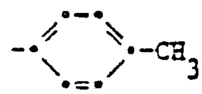 | rot |
| 31 | 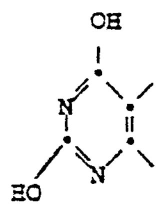 | - do - | 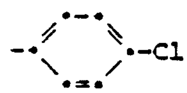 | gelb |
| 32 | 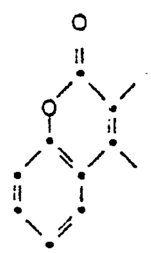 | $-CH_3$ | 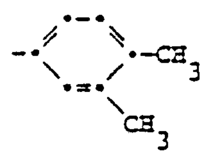 | scharlach |

Beispiel 33

28,5 g (0,1 Mol) 1-(Cyan-benzimidazolylmethylen)-3-imino-isoindolin, aus 1,3-Dimino-isoindolin und Cyanmethylbenzimidazol hergestellt, werden analog Beispiel 8 in Dimethylformamid (200 ml) bei Raumtemperatur mit Hydrazinhydrat (5,25 ml = 0,125 Mol) umgesetzt (Reaktionszeit 60 Minuten). Das Reaktionsprodukt wird anschliessend mit Isopropanol (650 ml) gefällt und wie üblich aufgearbeitet. Man erhält 24 g (80 % der Theorie) der Verbindung der Formel

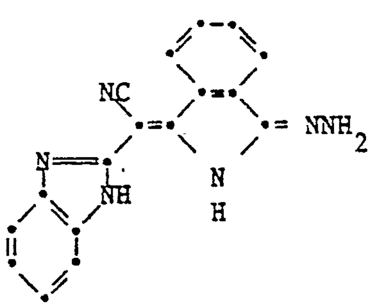

| | | | | | |
|---|---|---|---|---|---|
| Mikroanalyse : | | $C_{17}H_{12}N_6$ | | MG 300,3 | |
| gef. : | 67,99 % C | 4,03 % H | 27,99 % N | | |
| ber. : | 67,9 % C | 4,3 % H | 27,8 % N | | |

Beispiel 34

Arbeitet man analog Beispiel 1 mit 4-Chlor-1,3-diiminoisoindolin anstatt 1,3-Diiminoisoindolin unter sonst gleichen Bedingungen, so erhält man 3,87 g (57,3 % d. Theorie) des folgenden Zwischenproduktes

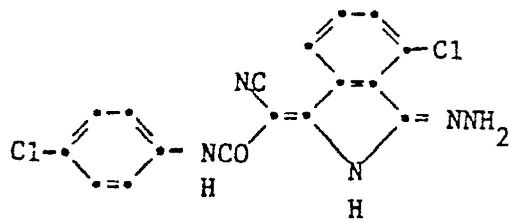

Beispiel 35

Verwendet man im Beispiel 9 anstelle des nach Beispiel 1 erhaltenen 1-(Cyano-p-chlorphenyl-carbamoylmethylen)-3-hydrazino-isoindolins das aus Beispiel 33 stammende Hydrazinoisoindolin-Derivat, so erhält man unter sonst identischen Reaktionsbedingungen einen roten Metallkomplex der folgenden Formel (nur eine der möglichen isomeren bzw. tautomeren Formen wurde berücksichtigt) :

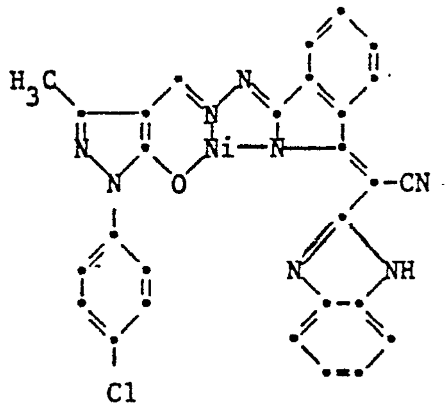

| | | | | | |
|---|---|---|---|---|---|
| Mikroanalyse : | $C_{28}H_{17}ClN_8ONi$ | | Mol.-Gew. 575,7 | | |
| ber. : | 58,42 % C | 2,98 % H | 19,46 % N | 6,16 % Cl | 10,2 % Ni |
| gef. : | 57,8 % C | 3,3 % H | 19,7 % N | 5,6 % Cl | 10,6 % Ni |

Mit dem obigen Ni-Komplex kann man Lacke, Kunststoffe und PVC in reinen roten Tönen von ausgezeichneten Pigmentechtheiten färben.

Beispiel 36

1,87 g (0,006 Mol) 1-p-Chlorphenyl-3-methyl-4-anilino-methylen-5-pyrazolen und 1,57 g (0,0063 Mol) Nickelacetat. 4 $H_2O$ werden in 60 ml Dimethylformamid bei 60-70 °C gelöst und anschliessend unter Rühren mit 2,02 g (0,006 Mol) des nach Beispiel 34 erhaltenen Zwischenproduktes versetzt. Das Gemisch wird auf 110-115 °C erhitzt und bei dieser Temperatur 2 Stunden lang weitergerührt. Danach wird auf 80 °C abgekühlt und der resultierende Metallkomplex analog Beispiel 9 aufgearbeitet. Man erhält 3,5 g (95 % der Theorie) eines roten Metallkomplexes der folgenden Formel (nur eine der möglichen isomeren bzw. tautomeren Formen wurde berücksichtigt)

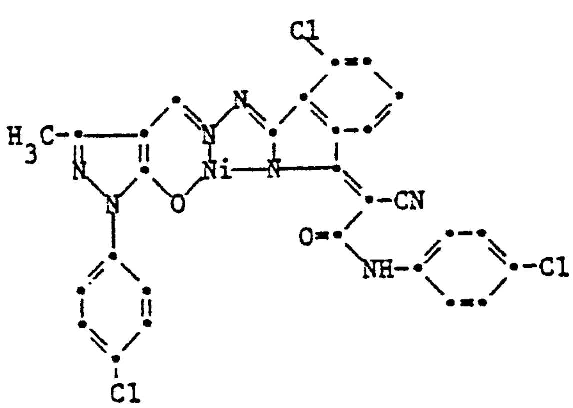

Mikroanalyse : $C_{28}H_{16}Cl_3N_7O_2Ni$ MG 647,3

| | | | | | |
|---|---|---|---|---|---|
| ber. : | 54,85 % C | 2,80 % H | 15,99 % Cl | 11,57 % N | 9,58 % Ni |
| gef. : | 54,6 % C | 3,0 % H | 16,3 % Cl | 11,4 % N | 9,29 % Ni |

Der obige Metallkomplex färbt Kunststoffe und Lacke in roten Tönen von einem hohen Echtheitsniveau.

**Patentansprüche**

1. Verfahren zur Herstellung von 1-Hydrozono-isoindolinen der Formel

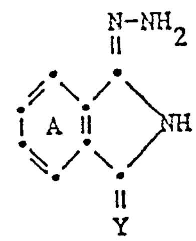

worin Y

a) einen Methinrest der Formel

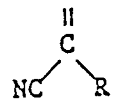

bedeutet, worin R für eine Alkoxycarbonyl-, Alkylcarbamoyl-, Carbamoyl- oder Sulfamoylgruppe, eine Benzylcarbamoylgruppe, oder eine Gruppe der Formel CONHB bedeutet, worin B einen Benzolrest bedeutet, der Halogenatome, Alkyl- oder Alkoxygruppen mit 1-6 C, Alkanoylamino-gruppen mit 2-6 C, Carbamoyl oder Trifluormethylgruppen, oder eine gegebenenfalls durch Halogenatome oder Alkylgruppen mit 1-4 C substituierte Phenoxy- oder Benzoylaminogruppe aufweisen kann, oder worin R für eine gegebenenfalls durch Chloratome substituierte Naphthylcarbamoylgruppe, eine Phenylsulfonylgruppe oder einen Rest der Formel

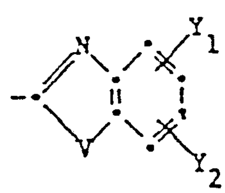

steht, worin V ein O- oder S-Atom oder eine Iminogruppe, $Y_1$ und $Y_2$ H- oder Halogenatome, Alkyl- oder Alkoxygruppen mit 1-4 C oder Nitrogruppen bedeuten oder

b) einen sich von einer heterocyclischen Verbindung mit aktiver Methylengruppe ableitenden Methinrest oder

c) einen sich von einem heterocyclischen aromatischen Amin ableitenden Iminrest bedeutet, und der Ring A nicht-wasserlöslichmachende Substituenten aufweisen kann, durch Umsetzung eines Iminoisoindolins der Formel

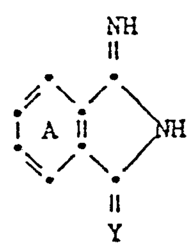

worin A und Y die oben angegebenen Bedeutungen haben mit Hydrazin oder einem Hydrazin abgebenden Mittel in einem polaren Lösungsmittel, dadurch gekennzeichnet, dass man die Umsetzung bei Temperaturen von höchstens 40 °C durchführt und das Reaktionsgemisch, sofern es eine aliphatische Carbonsäure enthält, letztere in nicht mehr als der doppelt molaren Menge, bezogen auf das Iminoisoindolin, vorhanden ist.

2. Verfahren gemäss Anspruch 1, dadurch gekennzeichnet, dass man die Reaktionen bei Temperaturen zwischen 10 und 40 °C durchführt.

**Claims**

1. A process for the preparation of a 1-hydrazinoisoindoline of the formula

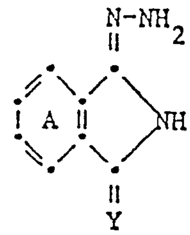

wherein Y is

a) a methine radical of the formula

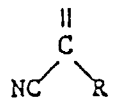

wherein R is an alkoxycarbonyl, alkylcarbamoyl, carbamoyl or sulfamoyl group, a benzylcarbamoyl group, or a group of the formula CONHB, wherein B is a radical of the benzene series which may contain halogen atoms, alkyl or alkoxy groups of 1 to 6 carbon atoms, alkanoylamino groups of 2 to 6 carbon atoms, carbamoyl or trifluoromethyl groups, or which may contain a phenoxy or benzoylamino group which is unsubstituted or substituted by halogen atoms or alkyl groups of 1 to 4 carbon atoms, or wherein R is a naphthylcarbamoyl group which is unsubstituted or substituted by chlorine atoms, or is a phenylsulfonyl group or a radical of the formula

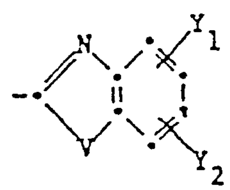

wherein V is an oxygen or sulfur atom or an imino group, $Y_1$ and $Y_2$ are hydrogen or halogen atoms, alkyl or alkoxy groups, each of 1 to 4 carbon atoms, or nitro groups, or

b) a methine radical derived from a heterocyclic compound containing an active methylene group, or

c) an imine radical derived from a heterocyclic aromatic amine, and the ring A may contain substituents which do not confer solubility in water, by reacting an iminoisoindoline of the formula

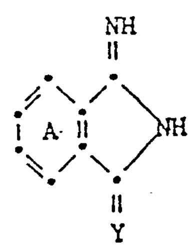

wherein A and Y are as defined above, with hydrazine or a hydrazine donor, in a polar solvent, wherein the reaction is carried out at a temperature of not more than 40 °C and, if the reaction mixture contains an aliphatic carboxylic acid, said carboxylic acid is present in not more than twice the molar amount, based on iminoisoindoline.

2. A process according to claim 1, wherein the reaction is carried out in the temperature range from 10° to 40 °C.

**Revendications**

1. Procédé pour préparer des hydrazono-1 iso-indolines répondant à la formule :

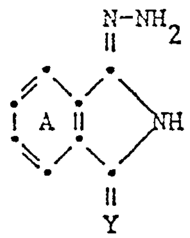

dans laquelle Y représente :

a) un radical méthinique de formule :

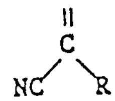

dans lequel R représente un radical alcoxycarbonyle, alkylcarbamoyle, carbamoyle ou sulfamoyle, un radical benzylcarbamoyle ou un radical -CONHB dans lequel B représente un radical benzénique éventuellement porteur d'atomes d'halogènes, de radicaux alkyles ou alcoxy en $C_1$-$C_6$, de radicaux alcanoylamino en $C_2$-$C_6$, de radicaux carbamoyles ou trifluorométhyles ou encore d'un radical phénoxy ou benzoylamino éventuellement porteur d'atomes d'halogènes ou d'alkyles en $C_1$-$C_4$, ou R représente un radical naphtylcarbamoyle éventuellement porteur d'atomes de chlore, un radical phénylsulfonyle ou un radical de formule :

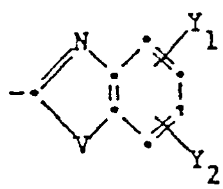

dans lequel V représente un atome O ou S ou un radical imino, $Y_1$ et $Y_2$ représentent des atomes d'hydrogène ou d'halogène, des radicaux alkyles ou alcoxy en $C_1$-$C_4$ ou des radicaux nitro, ou

b) un radical méthine dérivant d'un composé hétérocyclique à radical méthylène actif, ou

c) un radical imino dérivant d'une amine aromatique hétérocyclique, et le noyau A peut porter des substituants non hydrosolubilisants, par réaction d'une imino-iso-indoline répondant à la formule :

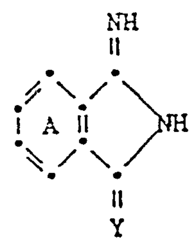

dans laquelle A et Y ont les significations précédemment données, avec l'hydrazine ou avec un agent cédant de l'hydrazine, dans un solvant polaire, procédé caractérisé en ce qu'on effectue la réaction à des températures d'au plus 40 °C et en ce que le mélange réactionnel, lorsqu'il renferme un acide carboxylique aliphatique, n'en contient pas plus du double de la quantité molaire par rapport à l'imino-iso-indoline.

2. Procédé selon la revendication 1 caractérisé en ce qu'on effectue les réactions à des températures comprises entre 10 et 40 °C.